# EUROPÄISCHE PATENTSCHRIFT

(11) **EP 2 730 293 B1**
(45) Veröffentlichungstag und Bekanntmachung des Hinweises auf die Patenterteilung: **22.07.2015**
(21) Anmeldenummer: 12192328.8
(22) Anmeldetag: 13.11.2012
(51) Int. Cl.: A61L 2/238, A61L 9/16, B01D 46/00, A62B 23/02, A61M 16/10

(54) **Gasfilter mit Kupfer zum Entfernen von Bakterien und Viren aus einem Gasvolumen**
Gas filter with copper for removing bacteria and viruses from a gas volume
Filtre à gaz avev cuivre destiné à éliminer les bactéries et virus d'un volume de gaz

(43) Veröffentlichungstag der Anmeldung: 14.05.2014
(73) Patentinhaber: Heyer Medical AG, 56130 Bad Ems (DE)
(72) Erfinder: Sascha, Tietz, 56412 Gackenbach (DE); Gertrud, Werner, 55424 Münster-Sarmsheim (DE)
(74) Vertreter: Schwan Schorer & Partner mbB

(56) Entgegenhaltungen:
- EP-A2- 1 946 638
- WO-A1-93/10896
- WO-A1-96/19645
- WO-A1-03/090873
- WO-A2-2008/036791
- DE-A1- 3 805 407
- GB-A- 353 409
- US-A1- 2010 287 897
- US-A1- 2012 285 459
- US-B1- 6 431 169
- DATABASE WPI Week 201101 17. November 2010 (2010-11-17) Thomson Scientific, London, GB; AN 2010-Q17753 XP002693818, & JP 4 581027 B1 (KODERA COPLON KK) 17. November 2010 (2010-11-17)

## Beschreibung

Die vorliegende Erfindung betrifft ein Gasfilter zum Entfernen von Bakterien und Viren aus einem Gasvolumen gemäß dem Oberbegriff von Anspruch 1 und eine Beatmungsgerät oder einem Anästhesiegerät mit einem erfindungsgemäßen Filter gemäß Anspruch 9.

Die EP 1 946 638 A2 offenbart einen Filter eines Kraftfahrzeugs der mit Kupferionen versehen ist. Die WO 2008/036791 A2 offenbart ein Beatmungsgerät mit einem mit Mangandioxid oder Kupferoxid oberflächenbeschichteten Filter, wobei diese Materialien eine katalytische Wirkung entfachen sollen, um Kohlenmonoxid auszufiltern. Die WO 93/10896 A1 offenbart einen Atemfilter, welcher 0 bis 20% Kupfer und 0 bis 20% Zink beinhaltet zur Adsorption von toxischen Gasen wie Arsenwasserstoff und Phosphin. Die GB 353,409 A offenbart, dass zur Sterilisation von Flüssigkeiten Silber-, Quecksilber- oder Kupfersalze Verwendung finden können. Die US 6,431,169 B1 offenbart einen Filter für ein Beatmungsgerät, der aus einem Sintermaterial aus Bronze besteht und der Reinigung eines Gases dient. Die US 2010/0287897 A1 beschreibt ein Filterelement aus einem gesinterten Pulver aus Kupfer oder einer Kupferlegierung, wobei diese Materialien die Korrosionsfestigkeit eines solchen Filters verbessern sollen. Die US 2012/0285459 A1 zeigt einen Filter zeigt mit mehreren Filterscheiben, die hintereinander angeordnet sind.

Anästhesie- und Beatmungsgeräte dienen der Zufuhr von Gasen zu einem Patienten (Mensch oder Tier). Dabei kann einerseits über ein Beatmungsgerät lediglich Umgebungsluft dem Patienten zugeführt werden, oder, im Falle einer Anästhesie, ein Gemisch von Sauerstoff oder Luft mit Anästhesiegasen. Zur Vereinfachung werden im Folgenden solche Gasgemische als "Luft" bezeichnet auch wenn es sich um ein Gasgemisch zur Anästhesie handeln könnte.

Um die Luft vom Anästhesie- bzw. Beatmungsgerät in die Lunge des Patienten zu führen, werden Schlauchsysteme verwendet, die wiederum mit einer Atemmaske auf Mund und Nase des Patienten, oder, im Falle einer Inkubation, mit einem Tubus, der beispielsweise mittels eines Ballonsystems in der Luftröhre des Patienten fixiert ist, verbunden. Das entsprechende Schlauchsystem wird nicht nur zur Zufuhr der Luft verwendet, sondern dient auch der Abfuhr von ausgeatmeter Atemluft, dient also der bidirektionalen Gasbewegung.

Um die Kontamination einerseits des Patienten, aber andererseits auch der Umgebung sowie der Geräte mit Bakterien und/oder Viren zu verhindern oder zumindest zu reduzieren, werden in bisher bekannten Schlauchsystemen Filter verwendet. Diese Filter sind im Wesentlichen mechanische Filter, haben also Filterelemente, welche Bakterien und Viren zurückhalten können. Die Wirksamkeit solcher bekannter Filterelemente gegen Kontamination mit Bakterien, Viren und Pilzen kann bei bestimmungsgemäßem Gebrauch 99,999 % erreichen.

Bei unsachgemäßem Gebrauch oder Nichteinhaltung der vorgeschriebenen Wechselintervalle kann es jedoch zu einer Kontamination von Atemluft führenden Teilen des Geräts in Größenordnung führen, die zu wesentlichen gesundheitlichen Risiken für die Patienten und gegebenenfalls auch für die Umgebung, also beispielsweise die Anwender, führenden können. Beispiele sind Krankenhausinfektionen (Nosokomiale Infektionen) oder die Infektionen mit multiresistenten Erregern (MRE).

Es ist daher Aufgabe der vorliegenden Erfindung, einen Gasfilter bzw. ein Verfahren zu schaffen zu schaffen, welcher bzw. welches die Effizienz des Schutzes gegen Kontamination erhöht und bestehende Hygienemaßnahmen ergänzt.

Diese Aufgabe wird gelöst durch einen Gasfilter gemäß dem Kennzeichen von Anspruch 1 und durch ein Beatmungs- oder Anästhesiegerät gemäß Anspruch 9.

Erfindungsgemäß ist ein Oberflächenbereich des Filterelements mit einer Kupferlegierung versehen. Es wurde im Rahmen der vorliegenden Erfindung festgestellt, dass eine Kontamination eines Gasvolumens reduziert wird, wenn das Gasvolumen an einem Oberflächenbereich des Filterelements, der eine entsprechende Kupferlegierung aufweist, entlanggeführt wird. Ein Filterelement kann dabei nur eine Oberfläche aus einer Kupferlegierung aufweisen, oder es kann vollständig aus der Kupferlegierung gefertigt sein. Somit bieten erfindungsgemäße Gasfilter den Vorteil, dass sie gegenüber bekannten Gasfiltern, die in Anästhesie- und/oder Beatmungsgeräten Verwendung finden, eine neue alternative oder ergänzende Wirkungsweise zur Dekontamination entfachen.

Die Kupferlegierung hat einen Kupferanteil von mindestens 70 % Gewichtsprozent, da so eine besonders effiziente Dekontaminierung erzielt werden kann.

Die Kupferlegierung weist weiterhin einen Mangananteil auf im Bereich von 15 bis 20 Gewichts%, was besonders vorteilhaft ist.

Die Kupferlegierung ist ferner mit einem Zinkanteil versehen, welcher in einem Bereich von 10 bis 15 Gewichts% liegt, wodurch die Wirkungsweise des Filterelements weiter verbessert wird.

Die Kupferlegierung beinhalten außerdem einen Aluminiumanteil in einem Bereich von 0,5 bis 2 Gewichts%.

Bevorzugte Ausgestaltungen der Erfindung ergeben sich aus den Unteransprüchen.

Vorzugsweise ist die Kupferlegierung so ausgebildet, dass sie eine toxische Wirkung auf die Bakterien und Viren entfaltet.

Zusätzlich können bei bevorzugten Ausführungsformen eines erfinderischen Gasfilters die Durchgangsöffnungen des Filterelements einen Querschnitt aufweisen, der mittels einer mechanischen Filterwirkung eine Entfernung von Bakterien und Viren bewirkt. Dies bietet den Vorteil, dass, neben der Dekontamination über die Toxizität der Kupferlegierung für Bakterien und Viren, diese auch mechanisch zurückgehalten werden.

Die Durchgangsöffnungen des Filterelements eines erfindungsgemäßen Gasfilters haben vorzugsweise einen durchschnittlichen Querschnitt von weniger als 1 µm, vorzugsweise von kleiner oder gleich 0,5 µm. Somit werden Viren und Bakterien mit einem Durchmesser ab 1 µm, bzw. ab 0,5 µm besonders effizient mechanisch zurückgehalten.

Bei weiteren bevorzugten Ausführungsformen weisen die der Durchgangsöffnung einen durchschnittlichen Querschnitt von mindestens 0,1 µm und maximal 0,5 µm auf. Die untere Bereichsgrenze gewährleistet, dass der Strömungswiderstand des Gasvolumens durch das Filterelement nicht zu groß wird. Ein zu großer Strömungswiderstand würde einen höheren Ausatemwiderstand für den Patienten bedeuten, was nicht wünschenswert ist.

Im Wesentlichen kann ein Gasfilter gemäß der vorliegenden Erfindung in drei unterschiedlichen Varianten (oder Kombinationen dieser Varianten) hergestellt sein. Bei jeder dieser Varianten kann der mittlere oder durchschnittliche Querschnitt der Durchgangsöffnung durch die Filterelemente so klein sein, dass er neben der toxischen Wirkung der Kupferlegierung Bakterien und Viren auch mechanisch zurückhält. Alternativ kann ein entsprechendes Filterelement auch Durchgangsöffnungen aufweisen, welche so dimensioniert sind, dass sie Bakterien und Viren zwar grundsätzlich durchlassen. Allerdings kann dann ein entsprechendes Filterelement durch den Strömungswiderstand die Verweildauer des Gasvolumens im Filterelement so erhöhen, dass die toxische Wirkung durch die Kupferlegierung ausreichend ist, um die gewünschte Dekontaminierung zu erreichen. Damit kann über die Länge der Durchgangsöffnungen bzw. die Länge des Filterelements (in Strömungsrichtung gesehen) und/oder wiederum die Dimensionierung der Durchgangsöffnung die Verweildauer des Gasvolumens und somit die Effizienz der Kontamination eingestellt werden. Bevorzugte Längen von Filterelementen sind daher 0,5 bis 5cm, insbesondere 0,75 bis 2cm.

Bei einer ersten Ausgestaltung eines erfindungsgemäßen Gasfilters ist das Filterelement plattenformig ausgebildet. Es handelt sich um eine plattenförmige runde oder eckige Scheibe und die Durchgangsöffnungen sind röhrenförmig, wobei die Röhren beginnend von einer ersten Plattenoberfläche durch das Filterelement hindurchgehen und auf der zweiten äußeren Plattenoberfläche enden.

Bei einer weiteren Grundform eines erfindungsgemäßen Gasfilters beinhaltet das Filterelement mehrere hintereinander angeordnete Scheiben oder Platten (ähnlich der vorbeschriebenen Plattenform) und mehrere solcher Scheiben oder Platten sind so hintereinander angeordnet und mit Durchgangsöffnungen versehen, dass die Durchgangsöffnungen von nachgeordneten Scheiben gegenüber Durchgangsöffnungen von vorher angeordneten Scheiben seitlich versetzt angeordnet sind. Da die Durchgangsöffnungen der unterschiedlichen Scheiben in einer Projektion entlang der Durchströmungsrichtung nicht aufeinander fallen, wird der Gasstrom durch den Gasfilter von Durchgangsöffnung zu Durchgangsöffnung mehrfach umgelenkt und die Verweildauer innerhalb des Gasfilters wird erhöht.

Bei einer dritten Variante kann ein erfindungsgemäßer Gasfilter ein Filterelement aus einem gesinterten Material aufweisen, welches die Kupferlegierung beinhalt. Dieses bedeutet, dass ein Grundmaterial, welches die Kupferlegierung aufweist, in entsprechenden Sinterformen geformt und ausgebacken wird, um eine gewünschte Form des Filterelements zu erhalten. Vorzugsweise weist somit ein entsprechendes Filterelement miteinander verbundene Körner auf, wobei vorzugsweise die Korngröße und somit die Größe von zwischen den Körnern angeordneten Hohlräumen solche Dimensionen aufweisen, dass Bakterien und Viren zurückgehalten werden. Vorzugsweise ist daher die mittlere Korngröße des gesinterten Materials in einem Bereich von 0,5 bis 10 µm im Durchmesser.

Bei einer weiteren Ausführungsform der Erfindung sind der Eingangsanschluss und der Ausgangsanschluss als ISO 5356-1 Anschlüsse ausgebildet, also insbesondere zur Verbindung mit Schläuchen von Anästhesiegeräten von 15 mm und 22 mm Durchmessern geeignet. Damit können erfindungsgemäße Gasfilter ohne weiteres in bekannte Patientenschlauchsysteme integriert werden.

Im Folgenden wird die Erfindung anhand der beigefügten Figuren 1 bis 3 näher erläutert.

Dabei zeigt:
- Fig. 1a:: einen Querschnitt durch einen Gasfilter mit einem Filterelement in Form eines Röhrenmodells,
- Fig. 1b:: eine Aufsicht auf das Filtermodell der Fig. 1a,
- Fig. 2a:: einen Querschnitt durch einen Gasfilter mit einem Filterelement in Form eines Scheibenmodells,
- Fig. 2b:: eine Aufsicht auf das Filtermodell der Fig. 2a,
- Fig. 3a:: einen Querschnitt durch einen Gasfilter mit einem Filterelement in Form eines Sintermodells,
- Fig. 3b:: eine Aufsicht auf das Filtermodell der Fig. 3a,

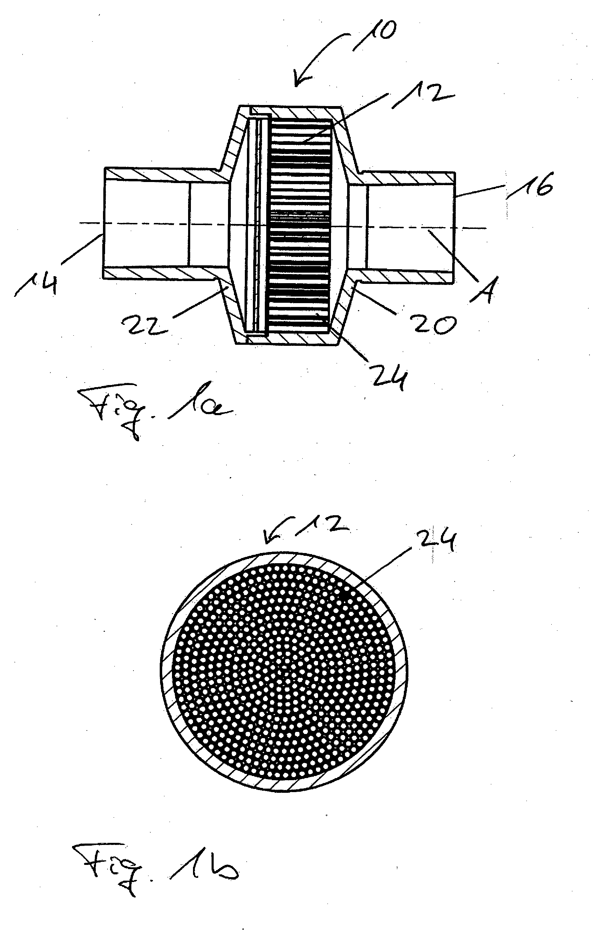

Die Figuren 1a bis 3a zeigen erfindungsgemäße Gasfilter 10, die mit Filterelementen 12 bestückt sind. Die jeweiligen Filterelemente bestehen erfindungsgemäß aus eine Kupferlegierung und sie sind in den Figuren 1b, 2b und 3b in einer Aufsicht dargestellt entlang der Achse in einer Durchströmungsrichtung (Achse A der Fig. 1a), also in Blickrichtung von links nach rechts in den Fig. 1a bis 3a.

Alle abgebildeten Gasfilter 10 weisen einen Eingangsanschluss 14 auf, sowie einen Ausgangsanschluss 16, die vorzugsweise als ISO 5356-1 Anschlüsse ausgebildet sind. So kann der Eingangsanschluss 14 als runder Anschlussstutzen mit einem Innendurchmesser von 22 mm ausgebildet sein, und der Ausgangsanschluss 16 als Anschlussstutzen mit einem Innendurchmesser von 15 mm und einem Außendurchmesser von 22 mm, die jeweils dazu vorgesehen sind, dass ebenfalls genormte Anschlussschläuche auf- oder eingesteckt werden können.

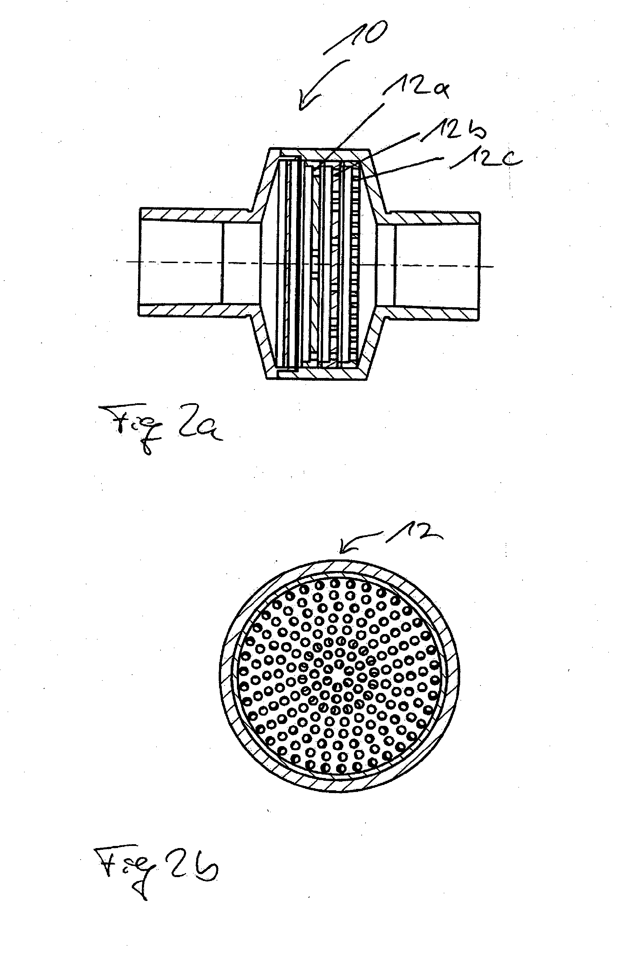

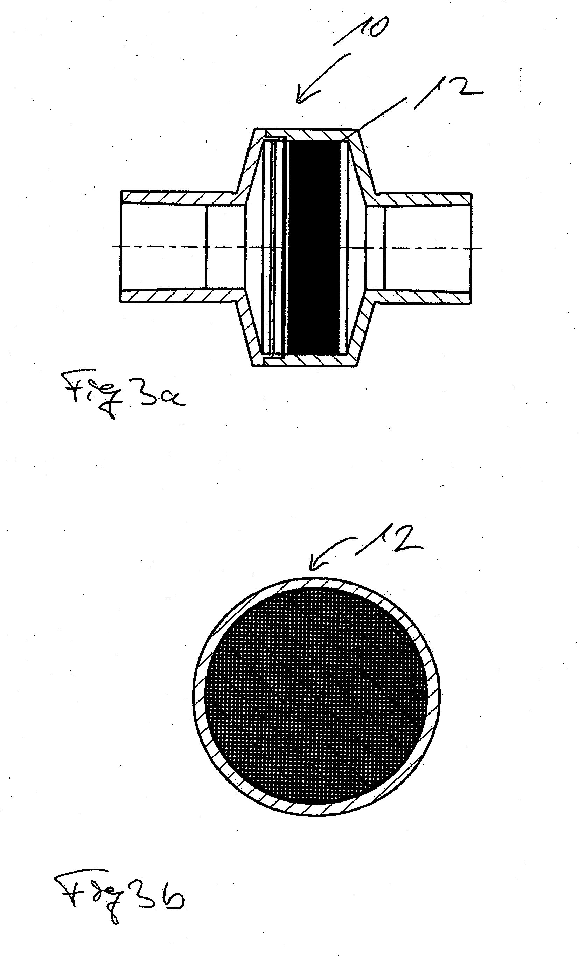

Die Gasfilter 10 der Fig. 1 bis 3 sind insbesondere dazu ausgebildet, zwischen einem Beatmungsgerät oder einem Anästhesiegerät (nicht dargestellt) auf der linken Seite und einer Schlauchverbindung zu einem Patienten auf der rechten Seite zwischengeschaltet zu werden. Somit wird durch die Filter 10 Luft in einer Durchströmung entlang der Achse A in Bildrichtung von links nach rechts dem Patienten zugeführt und bei einem Ausatmen des Patienten in die umgekehrte Richtung vom Patienten abgeführt. Ein Ventil zwischen Beatmungs- oder Anästhesiegerät kann dafür sorgen, dass ausgeatmete Atemluft beispielsweise über einen Kreislauf dem Anästhesiegerät wieder zugeführt oder im Falle eines Beatmungsgerätes in die Umgebung abgegeben wird. Dabei findet sowohl bei einer Strömungsrichtung von links nach rechts (also vom Eingangsanschluss 14 zum Ausgangsanschluss 16) eine Dekontamination der durchströmenden Luft durch das Filterelement 12 statt, als auch und auch bei der Rückströmung in die entgegengesetzte Richtung. Die Gasfilter 10 und die Filterelemente 12 der Fig. 1 bis 3 sind im Wesentlichen rotationssymmetrisch bezüglich der Achse A ausgebildet. Die dargestellten Gasfilter 10 weisen dabei ein erstes Gehäuseteil 20 und ein zweites Gehäuseteil 22 auf. Diese können beispielsweise mittels einer Schraubverbindung zusammengeschraubt werden, nachdem das Filterelement 12 im ersten Gehäuseteil 20 untergebracht wurde. Ein oder mehrere Dichtungen können zwischen den Gehäuseteilen 20 22 und dem Filterelement 12 vorgesehen sein (ebenso bei den Varianten der Fig. 2 und 3), die so angeordnet sind, dass die Luft nicht an dem Filterelement 12 vorbei strömen kann.

Die Fig. 1b zeigt das Filterelement 12 der Fig. 1a in einer Aufsicht mit Durchgangsöffnungen 24, die in diesem Fall röhrenförmig ausgebildet sind. Das Filterelement 12 ist daher ein sogenanntes "Röhrenmodell". Das Filterelement 12 ist in einer Aufsicht entlang der Achse A des Gasfilters 10 der Fig. 1a dargestellt. Die Durchgangsöffnung 24, die in der Fig. 1a in der Schnittbilddarstellung des Gasfilters 10 ebenfalls zu erkennen sind, sind entlang der Durchströmungsrichtung der Luft (also entlang der Achse A) ausgerichtet. Ein entsprechendes Filterelement 12 kann dabei (ebenso wie die Scheiben des weiter unten erläuterten Gasfilters 10 der Fig. 2) auf unterschiedliche Weise hergestellt werden, wie beispielsweise durch ein Strangpressverfahren. Die Durchgangsöffnungen 24 müssen dabei nicht notwendigerweise mechanisch Viren und Bakterien zurückhalten können, sondern können auch lediglich die Durchströmzeit von Atemluft durch das Filterelement 12, also die Verweildauer von Atemluft im Filterelement 12 so erhöhen, dass über die toxische Wirkung des Filterelements 12, welches aus der erfindungsgemäßen Kupferlegierung besteht, die gewünschte Dekontamination erzielt wird.

In Unterschied zum Gasfilter der Fig. 1a weist der Gasfilter der Fig. 2a nicht nur eine Scheibe auf, sondern das Filterelement 12 weist mehrere Scheiben 12a, 12b und 12c, die hintereinander angeordnet sind. Dabei weisen die Scheiben 12a, 12b, 12c unterschiedlich angeordnete Durchgangsöffnungen 24 auf. Unter unterschiedlicher Anordnung ist dabei zu verstehen, dass unterschiedlich gelochte Scheiben 12a, 12b und 12c Verwendung finden, oder dass Scheiben 12a, 12b und 12c zwar gleiche Muster von Durchgangslöchern 24 aufweisen, die Scheiben 12a, 12b und 12c aber innerhalb des Filters 10 gegeneinander verdreht eingebaut sind. Insgesamt wird dadurch erreicht, dass die Löcher von jeweils benachbarten Scheiben nicht in einer Projektion entlang der Achse A aufeinander fallen. Somit wird Luft, die durch das Filterelement 12 strömt, von Scheibe zu Scheibe umgelenkt, wodurch die Verweildauer (und damit wiederum die Dekontamination durch die Wirkung der Kupferlegierung des Filters 12) der Luft innerhalb des Gasfilters 10 erhöht wird.

Bei der Ausführungsform des Gasfilters 10 gemäß der Fig. 3 besteht das Filterelement 12 aus einem gesinterten Material. Somit kann nicht mehr von röhrenförmigen Durchgangslöchern gesprochen werden, sondern das Grundmaterial des Filterelements 12 (welches wie die oben besprochenen Ausführungsformen der Erfindung ebenfalls aus einer Kupferlegierung besteht) weist gewisse Korngrößen auf. Umgekehrt existieren zwischen benachbarten Körnern des Sintermaterials Hohlräume, durch die die Luft durch das Filterelement 12 strömen kann. Korngrößen und damit Hohlräume können dabei so gewählt werden, dass entweder durch einen entsprechenden Strömungswiderstand nur die Verweildauer der Atemluft im Filterelement 12 erhöht wird, eine mechanische Zurückhaltung von Bakterien und Viren aber nicht notwendigerweise gewährleistet wird. Die Dekontamination findet dann lediglich über die toxische Wirkung des gesinterten Kupferlegierungsmaterials statt. Alternativ können die Korngrößen und Hohlräume auch so klein gewählt werden, dass sie neben der chemischen Dekontamination bzw. toxischen Dekontamination auch eine Dekontamination über die mechanische Zurückhaltung der Viren und Bakterien bewirken.

## Patentansprüche

1. Gasfilter (10) zum Entfernen von Bakterien und Viren aus einem Gasvolumen, welches dazu vorgesehen ist, durch den Gasfilter der Lunge eines Lebewesens zugeführt zu werden, wobei der Gasfilter (10) ein Filterelement (12) aufweist mit einer Mehrzahl von Durchgangsöffnungen (24), die dazu ausgebildet sind, einen Eingangsanschluss (14) des Gasfilters (10) mit einem Ausgangsanschluss (16) des Gasfilters (10) in Fluidkommunikation zu verbinden,
**dadurch gekennzeichnet, dass**
ein Oberflächenbereich des Filterelements (12) eine Kupferlegierung aufweist, wobei die Kupferlegierung
einen Kupferanteil von mindestens 70 % aufweist.
einen Mangananteil in einem Bereich von 15 bis 20 %,
einen Zinkanteil in einem Bereich von 10 bis 15 %, und
einen Aluminiumanteil in einem Bereich von 0,5 bis 2 %.

2. Gasfilter (10) nach einem der vorhergehenden Ansprüche, **dadurch gekennzeichnet, dass** Durchgangsöffnungen (24) in dem Filterelement (12) einen Querschnitt aufweisen, der mittels einer mechanischen Filterwirkung eine Entfernung von Bakterien und Viren bewirkt.

3. Gasfilter (10) nach einem der vorhergehenden Ansprüche, **dadurch gekennzeichnet, dass** die Durchgangsöffnungen (24) in dem Filterelement (12) einen durchschnittlichen Querschnitt von weniger als 1 µm, vorzugsweise von kleiner / gleich 0,5 µm aufweisen, und der besonders bevorzugt in einem Bereich von 0,1 µm bis 0,5 µm liegt.

4. Gasfilter (10) nach einem der vorhergehenden Ansprüche, **dadurch gekennzeichnet, dass** das Filterelement (12) aus einem gesinterten Material besteht, welches die Kupferlegierung beinhaltet und vorzugsweise eine mittlere Korngröße von 0,5 bis 10 µm aufweist.

5. Gasfilter (10) nach einem der vorhergehenden Ansprüche 1 bis 3, **dadurch gekennzeichnet, dass** das Filterelement (12) plattenförmig ausgebildet ist und die Durchgangsöffnungen (24) in dem Filterelement (12) röhrenförmig ausgebildet sind, und die beiden äußeren Plattenoberflächen miteinander verbinden.

6. Gasfilter (10) nach einem der vorhergehenden Ansprüche 1 bis 3, **dadurch gekennzeichnet, dass** das Filterelement (12) mehrere Scheiben (12a, 12b, 12c) aufweist, die in einer Durchströmungsrichtung (A) hintereinander angeordnet sind und jeweils Durchgangsöffnungen (24) aufweisen, die so angeordnet sind, dass Durchgangsöffnungen (24) von nachgeordneten Scheiben gegenüber Durchgangsöffnungen von vorher angeordneten Scheiben seitlich versetzt angeordnet sind.

7. Gasfilter (10) nach einem der vorhergehenden Ansprüche, **dadurch gekennzeichnet, dass** ein Eingangsanschluss (14) und ein Ausgangsanschluss (16) als ISO 5356-1 Anschlüsse ausgebildet sind.

8. Gasfilter (10) nach einem der vorhergehenden Ansprüche, **dadurch gekennzeichnet, dass** ein Eingangsanschluss (14) als runder Anschlussstutzen mit einem Innendurchmesser von 22mm ausgebildet ist und ein Ausgangsanschluss (16) als runder Anschlussstutzen mit einem Innendurchmesser von 15mm und einem Außendurchmesser von 22mm.

9. Beatmungsgerät oder Anästhesieserät mit einem Gasfilters (10) gemäß einem der vorhergehenden Ansprüche, wobei der Gasfilter (10) in eine Schlauchverbindung zur Verbindung des Geräts und einem Patienten integriert ist.

## Claims

1. Gas filter (10) for removal of bacteria and viruses from a gas volume which is intended to be supplied through the gas filter to the lung of a living being, said gas filter (10) comprising a filter element (12) having a plurality of through-openings (24), that are adapted to connect an inlet port (14) of the gas filter (10) with an outlet port (16) of the gas filter (10) in fluid communication,
**characterized in that**
a surface area of the filter element (12) comprises a copper alloy, wherein the copper alloy comprises
a copper content of at least 70 %,
a manganese content in a range of 15 to 20 %,
a zink content in a range of 10 to 15 %, and
an aluminum content in a range of 0.5 to 2 %.

2. Gas filter (10) according to any of the preceding claims, **characterized in that** the through-openings (24) in the filter element (12) have a cross-section which effects a removal of bacteria and viruses by means of a mechanical filtering.

3. Gas filter (10) according to anyone of the preceding claims, **characterized in that** the through-openings (24) in the filter element (12) have an average cross-section of less than 1 µm, preferably of less than / equal to 0.5 µm, and more preferably in a range of 0.1 µm to 0.5 µm.

4. Gas filter (10) according to anyone of the preceding claims, **characterized in that** the filter element (12) consists of a sintered material which contains the copper alloy and preferably has an average grain size of 0.5 to 10 µm.

5. Gas filter (10) according to anyone of the preceding claims 1 to 3, **characterized in that** the filter element (12) is formed in plate-like manner and through-openings (24) in the filter element (12) are formed in a tubular manner and are connecting both outer surfaces of the plates with each other.

6. Gas filter (10) according to anyone of preceding claims 1 to 3, **characterized in that** a filter element (12) comprises a plurality of disks (12a, 12b, 12c) that are arranged one behind the other in a flow direction (A) and comprise through-openings (24), which are arranged such that through-openings (24) of subsequent disks are arranged with a lateral offset with respect to through-openings of preceding disks.

7. Gas filter (10) according to anyone of the preceding claims, **characterized in that** an inlet port (14) and an outlet port (16) are formed as ISO 5356-1 ports.

8. Gas filter (10) according to anyone of the preceding claims, **characterized in that** an inlet port (14) is formed as a round connecting piece with an inner diameter of 22 mm and an outlet port (16) is formed as a round connecting piece with an inner diameter of 15 mm and an outer diameter of 22 mm.

9. Respirator or apparatus for anesthesia comprising a gas filter (10) according to anyone of the preceding claims, wherein the gas filter (10) is integrated in a hose connection for connecting the apparatus and a patient.

## Revendications

1. Filtre à gaz (10) pour éliminer des bactéries et des virus d'un volume de gaz prévu pour être acheminé à travers le filtre à gaz jusqu'aux poumons d'un être vivant, le filtre à gaz (10) présentant un élément de filtre (12) avec une pluralité d'ouvertures de passage (24) qui sont réalisées de manière à relier, en communication fluidique, un raccord d'entrée (14) du filtre à gaz (10) à un raccord de sortie (16) du filtre à gaz (10),
**caractérisé en ce**
**qu'**une région de surface de l'élément de filtre (12) présente un alliage de cuivre, l'alliage de cuivre présentant
une proportion de cuivre d'au moins 70 %,
une proportion de manganèse dans une plage de 15 à 20 %,
une proportion de zinc dans une plage de 10 à 15 %, et
une proportion d'aluminium dans une plage de 0,5 à 2 %.

2. Filtre à gaz (10) selon la revendication précédente, **caractérisé en ce que** des ouvertures de passage (24) dans l'élément de filtre (12) présentent une section transversale qui, par un effet de filtre mécanique, produit une élimination des bactéries et des virus.

3. Filtre à gaz (10) selon l'une quelconque des revendications précédentes, **caractérisé en ce que** les ouvertures de passage (24) dans l'élément de filtre (12) présentent une section transversale moyenne inférieure à 1 µm, de préférence inférieure ou égale à 0,5 µm, et qui est particulièrement préférablement située dans une plage de 0,1 µm à 0,5 µm.

4. Filtre à gaz (10) selon l'une quelconque des revendications précédentes, **caractérisé en ce que** l'élément de filtre (12) se compose d'un matériau fritté, qui contient l'alliage de cuivre et présente de préférence une granulométrie moyenne de 0,5 à 10 µm.

5. Filtre à gaz (10) selon l'une quelconque des revendications précédentes 1 à 3, **caractérisé en ce que** l'élément de filtre (12) est réalisé en forme de plaque et des ouvertures de passage (24) dans l'élément de filtre (12) sont réalisées sous forme tubulaire, et relient l'une à l'autre les deux surfaces extérieures de la plaque.

6. Filtre à gaz (10) selon l'une quelconque des revendications précédentes 1 à 3, **caractérisé en ce que** l'élément de filtre (12) présente plusieurs disques (12a, 12b, 12c) qui sont disposés les uns derrière les autres dans une direction d'écoulement (A) et qui présentent chacun des ouvertures de passage (24) qui sont disposées de telle sorte que les ouvertures de passage (24) de disques disposés en aval soient disposées de manière décalée latéralement par rapport aux ouvertures de passage de disques disposés plus en amont.

7. Filtre à gaz (10) selon l'une quelconque des revendications précédentes, **caractérisé en ce qu'**un raccord d'entrée (14) et un raccord de sortie (16) sont réalisés sous forme de raccords selon la norme ISO 5356-1.

8. Filtre à gaz (10) selon l'une quelconque des revendications précédentes, **caractérisé en ce qu'**un raccord d'entrée (14) est réalisé sous forme de tubulure de raccordement ronde avec un diamètre intérieur de 22 mm et un raccord de sortie (16) est réalisé sous forme de tubulure de raccordement ronde avec un diamètre intérieur de 15 mm et un diamètre extérieur de 22 mm.

9. Appareil de respiration ou appareil d'anesthésie comprenant un filtre à gaz (10) selon l'une quelconque des revendications précédentes, dans lequel le filtre à gaz (10) est intégré dans une connexion tubulaire pour la connexion de l'appareil à un patient.
